# EUROPEAN PATENT SPECIFICATION

(11) **EP 3 858 313 B1**
(45) Date of publication and mention of the grant of the patent: **25.10.2023**
(21) Application number: 19886776.4
(22) Date of filing: 20.11.2019
(51) Int. Cl.: A61F 13/47, A61F 13/49, A61F 13/505, A61F 13/51, A61F 13/515

(54) **ABSORBENT PAD FOR UNDERWEIGHT BABY**
ABSORBIERENDES KISSEN FÜR UNTERGEWICHTIGEN SÄUGLING
GARNITURE ABSORBANTE POUR BÉBÉ EN INSUFFISANCE PONDÉRALE

(30) Priority: 22.11.2018 JP 2018219679
(43) Date of publication of application: 04.08.2021
(73) Proprietor: Unicharm Corporation, Ehime 799-0111 (JP)
(72) Inventor: TSUJII, Maki, Kanonji-shi, Kagawa 769-1602 (JP); YAMANAKA, Yasuhiro, Kanonji-shi, Kagawa 769-1602 (JP); SAKAGUCHI, Satoru, Kanonji-shi, Kagawa 769-1602 (JP)
(74) Representative: Staeger & Sperling Partnerschaftsgesellschaft mbB
(86) International application number: PCT/JP2019/045346
(87) International publication number: WO 2020/105656

(56) References cited:
- JP-A- 2009 082 484
- JP-A- 2009 082 484
- JP-A- 2012 071 060
- JP-A- 2012 071 160
- JP-A- 2015 208 624
- JP-A- 2015 208 624
- JP-A- 2018 068 721
- JP-A- 2018 068 721
- JP-A- 2019 208 961
- US-B2- 8 491 555

## Description

### FIELD

The present invention relates to an absorbent pad for a low-weight infant.

### BACKGROUND

Conventionally, a small diaper for an infant has been known as an absorbent article to be put on for a child whose body is small, such as infants.

For example, Patent Literature 1 discloses a tape-type disposable diaper that includes an absorbent body and a pair of fastening tapes, and a target wearer of which is mainly a low-weight infant of 3000 g (gram) or less.

### [CITATION LIST]

### [PATENT LITERATURE]

Patent Literature 1: Japanese Patent Application Publication No. 2016-202739. Further prior art in this technical field is disclosed in documents JP 2015-208624, JP 2009-082484 and JP 2018-068721.

### SUMMARY

### [TECHNICAL PROBLEM]

For the tape-type diaper as described in Patent Literature 1, a target wearer is intended to a low-weight infant whose weight is 3000 g or less.

However, in general, a low-weight infant has very delicate skin, and there is a risk that the skin is damaged due to contact with a hard member such as tape.

In addition, in a case where a target wearer is an extremely-low-weight infant whose weight is 1000 g or less, there is a risk that a normal absorbent article for a low-weight infant is unsuitable in size for and cannot be fitted to the wearer's body and/or does not absorb excrement properly.

The present invention was achieved in light of conventional problems such as that described above and an aspect of the present invention is to provide an absorbent article for a low-weight infant which is safe and has a good fitting property even in a case where the absorbent article is put on an extremely-low-weight infant.

### [SOLUTION TO PROBLEM]

The invention is defined by the features of claim 1. A main aspect of the present invention for achieving the above-described aspect is an absorbent pad for a low-weight infant having a longitudinal direction, a width direction, and a thickness direction, that are orthogonal to each other, the absorbent pad including: an absorbent body, a length of the absorbent pad in the longitudinal direction being 180 mm or less, a length of the absorbent pad in the width direction being less than 75 mm.

Features of the present invention other than the above will become clear by reading the description of the present specification with reference to the accompanying drawings.

### [ADVANTAGEOUS EFFECT OF THE INVENTION]

According to the present invention, it is possible to provide an absorbent article for a low-weight infant which is safe and has a good fitting property even in a case where the absorbent article is put on an extremely-low-weight infant.

### BRIEF DESCRIPTION OF DRAWINGS

FIG. 1 is a skin-side plan view of an absorbent pad 1 for a low-weight infant.
FIG. 2 is a non-skin-side plan view of the absorbent pad 1 for a low-weight infant.
FIG. 3 is a cross-sectional view taken along a line A-A of FIG. 1.
FIGS. 4A and 4B are diagrams showing a state in which the absorbent pad 1 according to the present embodiment is put on an extremely-low-weight infant.
FIGS. 5A and 5B are diagrams showing a state in which an absorbent pad 50 of a comparative example is put on an extremely-low-weight infant.
FIG. 6 is a diagram showing a state in which the absorbent pad 1 is placed on a skin-side surface of a diaper 100 in an unfolded and stretched state.
FIG. 7 is a schematic cross-sectional view taken along a line B-B of FIG. 6.
FIGS. 8A and 8B are diagrams for illustrating an aspect of use when the diaper 100 and the absorbent pad 1 are used in combination and put on an extremely-low-weight infant.

### DESCRIPTION OF EMBODIMENTS

At least following matters will become clear with description of this specification and attached drawings.

An absorbent pad for a low-weight infant having a longitudinal direction, a width direction, and a thickness direction, that are orthogonal to each other, the absorbent pad including: an absorbent body, a length of the absorbent pad in the longitudinal direction being 180 mm or less, a length of the absorbent pad in the width direction being less than 75 mm.

According to the absorbent pad for a low-weight infant, the length in the longitudinal direction is smaller, and this can make smaller the length of the portion of the absorbent pad which is laid on the back side of the extremely-low-weight infant (laying amount) when putting it on an extremely-low-weight infant.

Therefore, when laying the absorbent pad, it is possible to do it without lifting high the body (waist) of the extremely-low-weight infant, making it possible to reduce the physical burden on the extremely-low-weight infant.

In addition, the length in the width direction is small, and this makes the absorbent pad more likely to be fitted in the crotch (the groin) when the extremely-low-weight infant is sleeping with the legs bent strongly in an M shape, making it possible to enhance the fitting property.

In the absorbent pad for a low-weight infant, it is desirable that an elastic member is not provided in a region outside the absorbent body in the width direction.

According to the absorbent pad for a low-weight infant, the skin of an extremely-low-weight infant can be prevented from being injured, caused by the absorbent pad digging into the skin due to the stretchability exhibited by the elastic member.

Therefore, the safety when the absorbent pad is in use can be further enhanced.

In the absorbent pad for a low-weight infant, it is desirable that the absorbent pad does not have a folding line for folding the absorbent pad in the longitudinal direction.

According to the absorbent pad for a low-weight infant, since the folding line is not provided, crease lines are less likely to be formed, making less likely to generate a space between the skin of an extremely-low-weight infant and the absorbent pad when being put on the extremely-low-weight infant.

Thus, it is possible to easily fit the absorbent pad to the body of the extremely-low-weight infant.

In the absorbent pad for a low-weight infant, it is desirable that the absorbent pad further comprises a back sheet that is arranged on a non-skin side in the thickness direction with respect to the absorbent body, and that the back sheet is made of a nonwoven fabric.

According to the absorbent pad for a low-weight infant, even in a case where the back sheet comes into contact with the body of the extremely-low-weight infant when the absorbent pad is put on the extremely-low-weight infant, the back sheet being a flexible nonwoven fabric enables the skin of the extremely-low-weight infant to be less likely to be injured.

In the absorbent pad for a low-weight infant, it is desirable that the absorbent pad further comprises: a top sheet that is arranged on a skin side in the thickness direction with respect to the absorbent body, and a sealing portion in which at least a part of the top sheet is joined to the back sheet in the thickness direction.

According to the absorbent pad for a low-weight infant, the absorbent body is sandwiched between the top sheet and the back sheet, making it less likely for the shape of the absorbent body to collapse. Therefore, a good fitting property is more likely to be realized.

In addition, SAP (absorbent polymer) constituting the absorbent body is less likely to leak to the outside, making it possible to prevent the leaked SAP from adhering to the skin of the low-weight infant.

In the absorbent pad for a low-weight infant, it is desirable that the sealing portion is provided extending along an outer edge of the absorbent pad for a low-weight infant and spacing from the outer edge at a predetermined distance.

According to the absorbent pad for a low-weight infant, a dry edge is provided in an edge portion which is highly likely to come into contact with the body by being sandwiched between the legs of an extremely-low-weight infant when the absorbent pad is put on. This ensures the flexibility in the edge portion, making it possible to prevent the body of the wearer (extremely-low-weight infant) from being damaged.

In the absorbent pad for a low-weight infant, it is desirable that the absorbent body includes an absorbent polymer, and that the absorbent pad has a portion that is a part of an entire periphery of the sealing portion and that is not peeled off even when the absorbent polymer swells.

According to the absorbent pad for a low-weight infant, the sealing portion has a joining strength to the extent that, even when the SAP (absorbent polymer) included in the absorbent body swells, the sealing portion is not peeled off over the entire periphery. This suppresses the leaking of the SAP (absorbent polymer) to the outside of the sealing portion.

Therefore, it is possible to prevent the SAP from adhering to the skin of a low-weight infant.

In the absorbent pad for a low-weight infant, it is desirable that the absorbent body includes an absorbent polymer, and that a basis weight of the absorbent polymer in the absorbent body is 160 g/m2 or more.

According to the absorbent pad for a low-weight infant, even in a case of decreasing the area of the absorbent body in accordance with the body of an extremely-low-weight infant, excrement is absorbed reliably, and it is possible to suppress the leakage of the excrement.

As a result, for example, it is possible to suppress the leakage of the excrement to bed sheet, and to prevent causing the need to move the body of an extremely-low-weight infant due to the change of the sheet.

This makes less likely for the body of the extremely-low-weight infant to be subject to the burden.

In the absorbent pad for a low-weight infant, it is desirable that the absorbent pad does not include an adhesive portion for fixing the absorbent pad to a underwear of a wearer.

According to the absorbent pad for a low-weight infant, when the absorbent pad is put on, the absorbent pad can be easily laid between a low-weight infant and a mat or the like.

In addition, it is possible to prevent the adhesive portion from sticking to the legs of the low-weight infant who takes a posture in which the legs are strongly bent into an M shape.

In the absorbent pad for a low-weight infant, it is desirable that the absorbent pad further comprises: a top sheet that is arranged on a skin side in the thickness direction with respect to the absorbent body, and an intermediate sheet that is arranged between the absorbent body and the top sheet in the thickness direction.

According to the absorbent pad for a low-weight infant, the rigidity of the absorbent pad increases compared with the case where the intermediate sheet is not provided, and this can make it easier to lay the absorbent pad between a low-weight infant and a mat or the like.

In the absorbent pad for a low-weight infant, it is desirable that a density of the intermediate sheet is higher than a density of the top sheet.

According to the absorbent pad for a low-weight infant, increasing the density of the intermediate sheet increases the rigidity of the intermediate sheet, and also increases the rigidity of the absorbent pad.

This can make it easier to lay the absorbent pad between a low-weight infant and a mat or the like. In addition, the ability for drawing the excrement from the top sheet to the intermediate sheet is increased, and the rewet rate is decreased. This can improve the sense of comfort of the absorbent pad.

In the absorbent pad for a low-weight infant, it is desirable that the intermediate sheet includes a thermoplastic fiber.

According to the absorbent pad for a low-weight infant, in a process of joining the top sheet and the back sheet by heat sealing in the thickness direction, the intermediate sheet including the thermoplastic fiber is more likely to be fused between the top sheet and the back sheet, making it possible to increase the joining strength.

As a result, the joining strength of the sealing portion can be sufficiently increased.

In the absorbent pad for a low-weight infant, it is desirable that the absorbent pad is capable of being attached to and detached from a skin side of an absorbent main body of a disposable diaper, the disposable diaper having a lengthwise direction, a lateral direction, and a thickness direction, that are orthogonal to each other, the disposable diaper including: the absorbent main body including an absorbent core, a pair of leakproof walls that are provided on two lateral sides of the absorbent main body and are capable of rising up toward the skin side in the thickness direction, and a pair of fastening tapes that protrude outward beyond two lateral end portions of the absorbent main body.

According to the absorbent pad for a low-weight infant, in the case where the absorbent pad is used on the top of the diaper in an overlapped manner, only the absorbent pad that absorbed the excrement can be changed in excretion. This makes it possible to use the diaper repeatedly multiple times, making it possible to reduce the frequency of changing the diaper.

In the absorbent pad for a low-weight infant, it is desirable that a length of the absorbent pad for a low-weight infant in the longitudinal direction is smaller than a length of the absorbent core in the lengthwise direction.

According to the absorbent pad for a low-weight infant, even in the case where excrement leaks outside in the longitudinal direction of the absorbent pad, the excrement is absorbed by the absorbent core of the diaper laid below the absorbent pad (on non-skin side of the absorbent pad in the thickness direction), making it possible to suppress the leakage of the excrement.

In the absorbent pad for a low-weight infant, it is desirable that a length of the absorbent pad for a low-weight infant in the width direction is smaller than a lateral length between rising base points of the pair of leakproof walls, the rising base point being a point where each of the pair of leakproof walls starts to rise up toward the skin side.

According to the absorbent pad for a low-weight infant, the absorbent pad is inserted in and attached to a space between the absorbent main body and the leakproof walls of the diaper in the thickness direction, without compressing or folding the absorbent pad in the width direction.

Therefore, it is possible to use the absorbent pad in combination with the diaper without deteriorating the absorption performance of the absorbent pad.

In the absorbent pad for a low-weight infant, it is desirable that a length of the absorbent pad for a low-weight infant in the width direction is larger than a lateral length between peaks of the pair of leakproof walls.

According to the absorbent pad for a low-weight infant, the two widthwise end portions of the absorbent pad are held so as to be covered from the skin side by a tip end portion of the leakproof wall, making it less likely to displace the relative position of the absorbent pad with respect to the diaper.

This can make it easier to fit the absorbent pad to the body of a low-weight infant.

### Embodiment

An absorbent pad for a low-weight infant will be described as an absorbent article for a low-weight infant.

The "absorbent pad for a low-weight infant" is an absorbent pad for a low-weight infant whose weight is 3000 g or less.

Among the infants, the absorbent pad can be suitably applied to a low-birth-weight infant of less than 2500 g, a very-low-birth-weight infant of less than 1500 g, and an extremely-low-birth-weight infant of less than 1000 g (hereinafter, also referred to as "extremely-low-weight infant").

### Basic configuration of absorbent pad 1 for low-weight infant

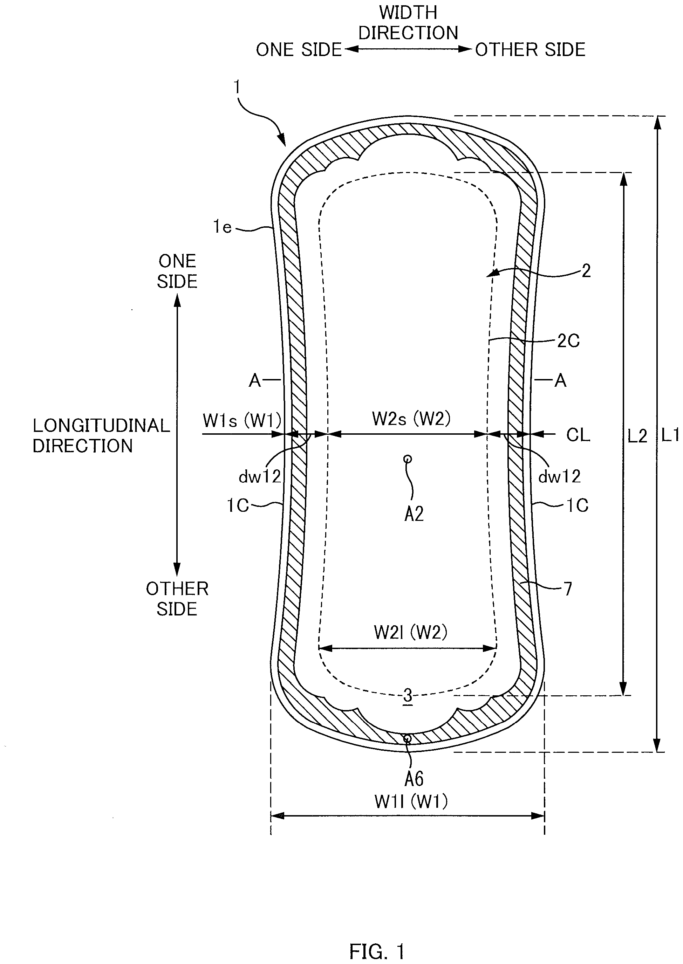

FIG. 1 is a skin-side plan view of an absorbent pad 1 for a low-weight infant (hereinafter, also referred to as "absorbent pad 1").

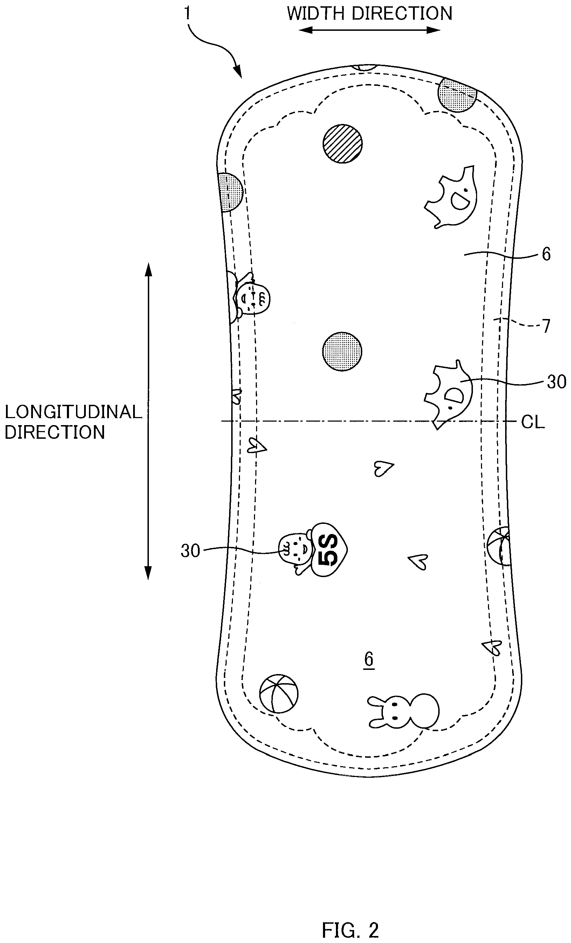

FIG. 2 is a non-skin-side plan view of the absorbent pad 1.

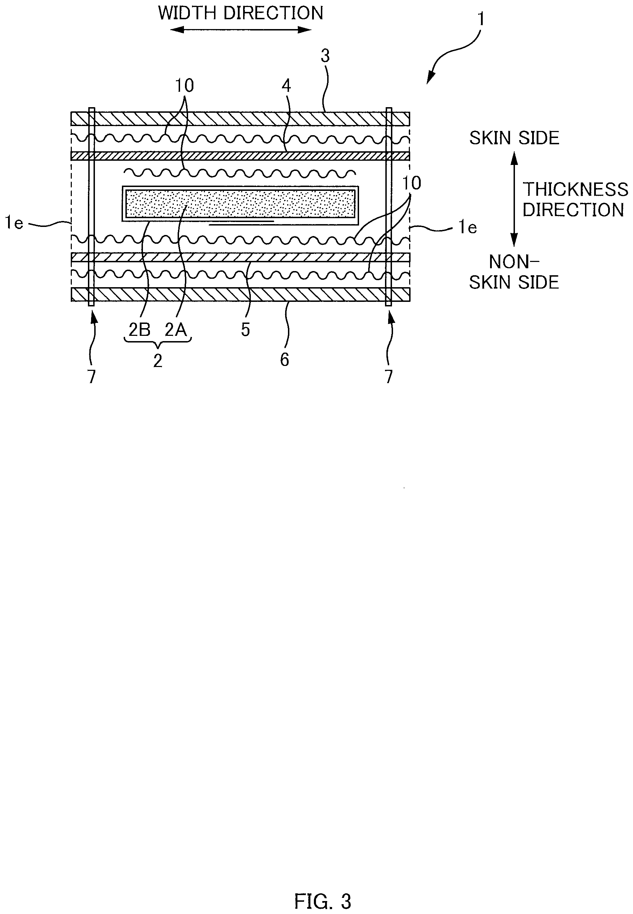

FIG. 3 is a schematic cross-sectional view taken along a line A-A of FIG. 1.

The absorbent pad 1 is a so-called flat type disposable diaper, which has a substantially rectangular shape (substantially oval shape) in a plan view, and has a longitudinal direction, a width direction, and a thickness direction, which are orthogonal to each other.

In the thickness direction, a side to be in contact with the wearer's body is called a skin side, and the opposite side is called a non-skin side.

As shown in FIG. 3, the absorbent pad 1 includes: an absorbent body 2; a top sheet 3 that is arranged on a skin side with respect to the absorbent body 2; an intermediate sheet 4 that is arranged between the absorbent body 2 and the top sheet 3; a back sheet 6 that is arranged on a non-skin side with respect to the absorbent body 2; and a leakproof sheet 5 that is arranged between the absorbent body 2 and the back sheet 6.

In addition, the materials adjacent to each other in the thickness direction are joined with an adhesive 10.

The absorbent body 2 has polymer absorbents (absorbent polymer superabsorbentpolymer, hereinafter, also referred to as "SAP") and liquid-absorbent fibers such as pulp fiber, and is formed in a longitudinally-elongated, substantially rectangular shape (substantially oval shape) as shown by the dashed line in FIG. 1 in the present embodiment.

The liquid-absorbent fiber (absorbent core 2A) containing SAP may be covered with a liquid-permeable core-wrapping sheet 2B as shown in FIG. 3, or does not have to be covered therewith. In addition, the configuration of the absorbent body 2 is not limited to the above. The examples thereof include a SAP sheet in which a SAP layer is attached to a hydrophilic sheet, an air-laid sheet in which liquid-absorbent fibers are formed into a sheet by an air-laid method, and the like.

The top sheet 3 is a liquid-permeable sheet member that is arranged farthest on the skin side in the thickness direction of the absorbent pad 1, and is a member that comes into direct contact with the skin of a wearer (low-weight infant) when the absorbent pad 1 is put on.

Therefore, it is desirable that the top sheet 3 is a sheet member that is as flexible as possible and has a soft texture.

Examples of the sheet member constituting the top sheet 3 of the present embodiment include an air-through nonwoven fabric and the like.

The intermediate sheet 4 is a liquid-permeable sheet member that is arranged between the absorbent body 2 and the top sheet 3 in the thickness direction of the absorbent pad 1.

Providing the intermediate sheet 4 increases the rigidity of the absorbent pad 1, making it easier to lay the absorbent pad 1 between a low-weight infant and a mat or the like.

Since the intermediate sheet 4 does not come into direct contact with the skin of a low-weight infant, the skin of the low-weight infant is less likely to be damaged even when the absorbent pad has high rigidity.

Examples of the sheet member constituting the intermediate sheet 4 of the present embodiment include a spunbond nonwoven fabric, an air-laid nonwoven fabric, tissue and the like.

However, the absorbent pad 1 does not have to include the intermediate sheet 40 necessarily.

The leakproof sheet 5 is a liquid-impermeable sheet member arranged on the non-skin side in the thickness direction of the absorbent pad 1 with respect to the absorbent body 2.

Providing the leakproof sheet 5 makes it possible to prevent the liquid such as urine absorbed by the absorbent body 2 from permeating into the clothing side (non-skin side) of the wearer.

Examples of the sheet member constituting the intermediate sheet 4 of the present embodiment include a resin film made of polyethylene and/or polypropylene.

The back sheet 6 is a sheet member that is arranged on the non-skin side of the leakproof sheet 5 in the thickness direction of the absorbent pad 1, and is a member (exterior sheet) constituting the exterior of the absorbent pad 1.

When the absorbent pad 1 is put on, the back sheet 6 is also likely to come into direct contact with the skin of the wearer (low-weight infant). Accordingly, it is desirable that the sheet member is as flexible as possible, like the top sheet 3.

Examples of the sheet member constituting the back sheet 6 of the present embodiment include an air-through nonwoven fabric and the like.

In the absorbent pad 1, design graphics 30 such as characters and graphics as shown in FIG. 2 are printed on a non-skin-side surface of the leakproof sheet 5, and are visually recognizable from the non-skin side of the absorbent pad 1 through the back sheet 6.

However, the present invention is not limited thereto, and the design graphic 30 may be printed on the skin-side surface of the leakproof sheet 5 or on the skin-side surface or the non-skin-side surface of the back sheet 6.

In the absorbent pad 1 of the present embodiment, both the top sheet 3 arranged farthest on the skin side and the back sheet 6 arranged farthest on the non-skin side are made of a nonwoven fabric, and therefore there is a risk that it is difficult to distinguish between the skin-side surface and the non-skin-side surface if the appearances of the top sheet 3 and the back sheet 6 are the same (for example, plain and the same color).

That is, there is a risk that the absorbent pad 1 is used upside down.

Accordingly, the design graphics 30 visually recognizable from the non-skin side makes the appearances of the back sheet 6 and the top sheet 3 different from each other.

This allows the user to visually recognize that the back sheet 6 is on the non-skin side, making it possible to prevent the absorbent pad 1 from being used upside down.

The design graphics 30 may include information regarding the size of the absorbent pad 1 and information that they are on the non-skin-side surface.

In addition, in the absorbent pad 1, a sealing portion 7 is provided in an outer peripheral edge portion, outside the absorbent body 2 in the longitudinal direction and the width direction.

In the sealing portion 7, the top sheet 3, the intermediate sheet 4, the leakproof sheet 5, and the back sheet 6 are welded and integrated by heat sealing.

The sealing portion 7 of the present embodiment is formed by arranging side-by-side a plurality of dot-shaped welded portions in the region shown by the shaded portion in FIG. 1.

That is, the sheet members are joined so as to surround the periphery of the absorbent body 2 along the edge portion of the absorbent pad 1.

However, the sealing portion 7 may be formed by a linear welded portion or a welded portion having another shape.

In the present embodiment, the top sheet 3, the intermediate sheet 4, the leakproof sheet 5, and the back sheet 6 all are sheet members having an identical shape and size. As shown in FIG. 3, the sheets are joined to each other in the thickness direction by the sealing portion 7.

However, the intermediate sheet 4 and the leakproof sheet 5 may be smaller than the top sheet 3 and the back sheet 6 constituting the exterior of the absorbent pad 1.

### Aspect of use of absorbent pad 1

Hereinafter, an aspect when the absorbent pad 1 is put on a low-weight infant (extremely-low-weight infant) will be described.

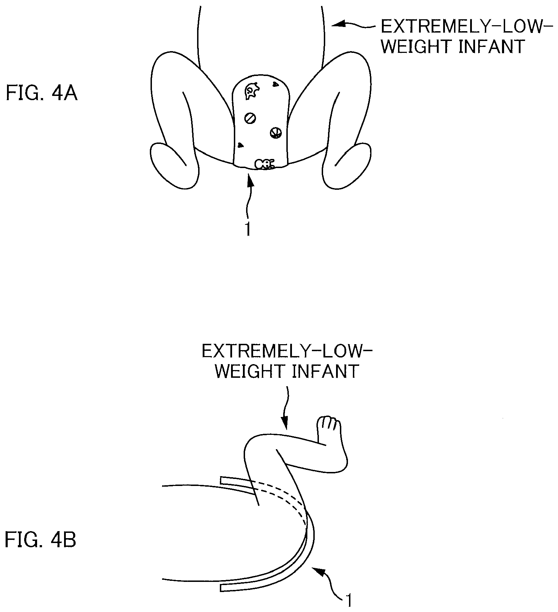

FIGS. 4A and 4B are diagrams showing a state in which the absorbent pad 1 according to the present embodiment is put on an extremely-low-weight infant.

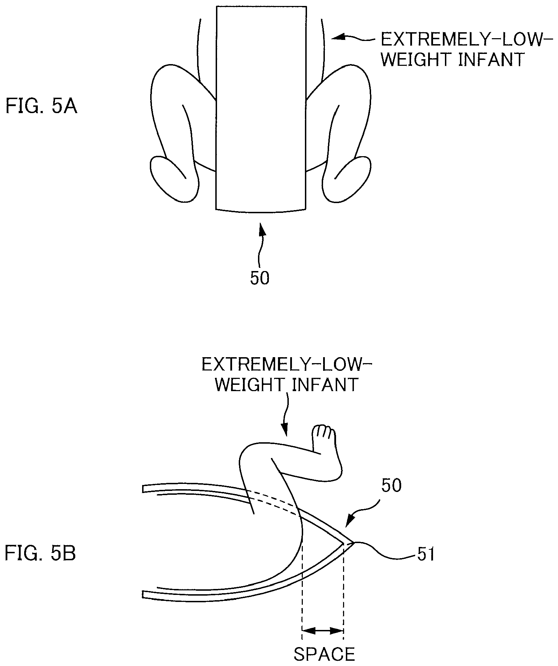

FIGS. 5A and 5B are diagrams showing a state in which an absorbent pad 50 of a comparative example is put on an extremely-low-weight infant.

FIGS. 4A and 5A show a state in which the lower half of the body of the extremely-low-weight infant viewed from the stomach-side, and FIGS. 4B and 5B show a state in which the lower half of the body of the extremely-low-weight infant viewed from the side.

A low-weight infant is generally raised in an incubator in a hospital and the like.

In the incubator, in order to keep the low-weight infant stable, the infant often sleeps on a mat and in a posture when in the womb (a posture in which the back is curved in a C shape and the legs are strongly bent in an M shape).

When changing the diaper (absorbent pad 1) of the low-weight infant, after the used diaper is removed from between the body of the low-weight infant and the mat, a new diaper is inserted between the body of the low-weight infant and the mat.

Among low-weight infants, an extremely-low-weight infant whose weight is less than 1000 g has extremely weak skin.

Accordingly, for the extremely-low-weight infant, the absorbent pad 1 having no fastening tape is suitably used.

This is because when changing the absorbent pad 1, there is no insertion and/or removing of a highly-rigid fastening tape between the body of the extremely-low-weight infant and the mat, and therefore it is possible to prevent the skin of the extremely-low-weight infant from being injured by the fastening tape.

In addition, the absorbent pad 1 is easier to put on and take off than a tape type diaper.

This makes it possible to change the absorbent pad 1 without unnecessarily moving the body of the extremely-low-weight infant, making it possible to keep the extremely-low-weight infant stable and to reduce the burden on the nurse or the like.

In addition, the extremely-low-weight infant or the extremely-low-weight infant often has medical devices such as tubes attached thereto, and/or is often subjected to phototherapy treatment.

Using the absorbent pad 1 can increase the exposed area of the skin of the low-weight infant.

This makes it easy to attach a medical device to the low-weight infant, and makes it possible to increase the irradiation area of phototherapy treatment. In this way, the absorbent pad can be used while suppressing the interruption of medical treatment.

In the present embodiment, in the state where the absorbent pad 1 is stretched in the longitudinal direction and in the width direction (see FIG. 1), a length (product length) L1 of the absorbent pad 1 in the longitudinal direction is 180 mm or less, and a length of the absorbent pad 1 in the width direction (product width) W1 is less than 75 mm (L1 ≤ 180 mm, W1 < 75 mm).

The length from the navel to the waist portion on the back side of the extremely-low-weight infant is about 140 mm.

Therefore, making the product length L1 as small as possible to 180 mm or less as described above can decrease the length (laying amount) of a portion of the absorbent pad 1 which is laid underneath on the back side of the extremely-low-weight infant (see FIG. 4B).

Accordingly, when laying the absorbent pad 1, it is possible to do it without lifting high the body (waist) of the extremely-low-weight infant or shifting the position of the body significantly.

Thus, it is possible to reduce the physical burden on the body of the extremely-low-weight infant.

In addition, on the stomach side of the extremely-low-weight infant, excessive coverage of the skin is suppressed (see FIG. 4A), and thus, medical treatment is less likely to be interrupted.

In addition, in the present embodiment, setting the product width W1 to less than 75 mm makes the absorbent pad 1 be comfortably fitted in the crotch (the groin) of the extremely-low-weight infant in a state where the extremely-low-weight infant is sleeping in the posture in which the legs are strongly bent into an M shape as shown in FIG. 4A.

Thus, it is possible to prevent the widthwise edge portion of the absorbent pad 1 from damaging the skin of the extremely-low-weight infant or from making it difficult for the infant to take the posture in which the legs are strongly bent into an M shape.

In addition, the absorbent pad 1 can be firmly fitted to the crotch portion of the extremely-low-weight infant.

Therefore, it is possible to reduce the burden on the body of the low-weight infant.

On the other hand, an absorbent pad 50 of comparative example is a product conventionally used for a low-weight infant, and is a substantially rectangular absorbent pad having a product length L50 larger than 180 mm and a product width W50 of 75 mm or more.

In the case where the absorbent pad 50 of comparative example is to be put on an extremely-low-weight infant, the product length L50 is too long for the body of the extremely-low-weight infant, and as shown in FIG. 5A, the absorbent pad 50 may cover not only the crotch but also to an area from the waist to the chest.

That is, the exposed area of the skin of the extremely-low-weight infant becomes small, causing a risk of interrupting medical treatment.

In addition, the product width W50 is too wide for the body of an extremely-low-weight infant, and the absorbent pad 50 is less likely to be fitted in the groin as shown in FIG. 5B.

In such a case, there is a high possibility that the absorbent pad 50 damages the skin of an extremely-low-weight infant or that the absorbent pad 50 makes less likely for the infant to take a posture in which the legs are strongly bent into an M shape. Accordingly, it is difficult to secure sufficient safety.

In addition, in the case where the absorbent pad 50 of comparative example is put on, the absorbent pad is not fitted to the body of the extremely-low-weight infant, so that the displacement of the absorbent pad or the leakage of excrement is likely to occur.

In this case, it is necessary to frequently correct the position of the absorbent pad 50 or change the absorbent pad 50, which increases the frequency of moving the body of the extremely-low-weight infant.

In other words, it becomes difficult to realize so-called "minimal handling" in which the frequency of touching the extremely-low-weight infant is reduced.

In the absorbent pad 1 of the present embodiment, appropriately adjusting the product length L1 and the product width W1 according to the body of the extremely-low-weight infant (L1 ≤ 180 mm, W1 < 75 mm) makes it possible to improve the safety and the fitting property when putting on the absorbent pad, compared with the conventional absorbent pad (for example, the absorbent pad 50 of comparative example).

In addition, as shown in FIGS. 1 to 3, the absorbent pad 1 is not provided with an elastic member having stretchability.

In particular, the elastic member is not provided in a region outside the absorbent body 2 in the width direction.

To the conventional absorbent pad, an elastic member (e.g., an elastic string) that is stretched and contracted in the longitudinal direction is provided on the two widthwise sides of the absorbent body, in order to enhance the fitting property around the wearer's legs and to make the leakproof walls stand up.

However, in the case where the absorbent pad is put on the extremely-low-weight infant, the absorbent pad having such an elastic member has a risk that it digs into the body of the extremely-low-weight infant due to the stretchability exhibited by the elastic member, injuring the infant's skin. Therefore, in the present embodiment, not providing the elastic member in the region outside in the width direction with respect to the absorbent body 2 prevents the skin of the extremely-low-weight infant from being injured, further enhancing the safety.

In addition, the absorbent pad 1 is not provided with a folding line for folding the absorbent pad 1 in the longitudinal direction.

Generally, in a product having a long product length (length in the longitudinal direction) such as the absorbent pad 50 of comparative example, it is common to fold the product in the longitudinal direction when packing and distribute it to the market in a compact shape as much as possible.

For example, by folding the product in two at the longitudinal center and packaging the product in half the product length, it is possible to facilitate handling during storage and transportation.

However, when such a folding line is formed, there is a risk that the absorbent pad 50 has a crease at the folding line, deteriorating the fitting property when the absorbent pad is put on the extremely-low-weight infant.

For example, in FIG. 5B, the crease is formed in the absorbent pad 50 based on a folding line 51 extending along the width direction, and the absorbent pad 50 is folded along the folding line 51 at an acute angle, generating a space between the wearer's crotch and the absorbent pad, and making it more likely for the fitting property to deteriorate.

In particular, in the extremely-low-weight infant whose body is small, the space becomes large and the deterioration of the fitting property becomes serious.

In contrast, in the absorbent pad 1 of the present embodiment, since the folding line is not provided, a crease is less likely to be formed.

Therefore, when the absorbent pad is put on the extremely-low-weight infant, it is easy to fit the absorbent pad 1 firmly along the roundness of the body as shown in FIG. 4B, and it is possible to prevent occurrence of a space.

Further, as described above, the back sheet 6 constituting the exterior of the absorbent pad 1 is formed of a flexible sheet member such as a nonwoven fabric.

As a result, even in the case where the back sheet 6 comes into contact with the groin of the extremely-low-weight infant when the absorbent pad 1 is put on, it is possible to prevent the skin of the extremely-low-weight infant from being injured.

In addition, since the top sheet 3 and the back sheet 6 are joined to each other by the sealing portion 7, the shape of the absorbent body 2 sandwiched between the top sheet 3 and the back sheet 6 does not easily collapse, and a good fitting property is more likely to be realized even for a wearer with a small body like an extremely-low-weight infant.

Further, the sealing portion 7 is formed by heat sealing means such as heat sealer, the joining strength is larger than that in the case of joining the sheets using an adhesive such as a hot-melt adhesive.

Accordingly, even when the absorbent body 2 absorbs excrement such as urine and swells, the sealing portion 7 is difficult to be separated (difficult to peel off), and the leakage of the SAP included in the absorbent body 2 to the outside is suppressed.

As a result, it is possible to prevent the leaked SAP from adhering to the skin of the low-weight infant.

The product length L1 and the product width W1 of the absorbent pad 1 are smaller than those of the conventional product. Accordingly, the volume in which the absorbent body 2 can be accommondated when the SAP (absorbent polymer) included in the absorbent body 2 swells is small, and a force is more likely to act in a direction of pulling apart the sealing portion 7.

Therefore, in the present embodiment, the joining strength of the sealing portion 7 is sufficiently increased to the extend that, even when the SAP (absorbent polymer) swells, the sealing portion 7 does not peel off over the entire periphery. This suppresses the leakage of the absorbent body 2 (such as SAP) to the outside.

The joining strength of the sealing portion 7 is verified by conducting the following experiment.

First, the absorbent pad 1 is placed in a flat-bottomed container with the skin-side surface facing up. The container to be used includes a bottom face having a width on which the absorbent body 2 can be placed completely flat, and a wall surface having a height (depth) of about 10 cm.

Then, a physiological saline solution is poured in the container to a depth of 5 cm in an environment maintained at about 37°C.

Then, the appearance of the sealing portion 7 of the absorbent pad is observed at intervals of 5 minutes, and it is confirmed whether or not peeling off occurs, leaking from the absorbent body 2 (such as SAP).

Consequently, in the experiment for 60 minutes, a portion in which the sealing portion 7 is peeled off has not been found, and the absorbent body 2 (such as SAP) have not leaked.

In the present embodiment, the basis weight of SAP (absorbent polymer) included in the absorbent body 2 is 160g/m² or more.

It is preferable that the SAP is dispersed substantially evenly on the entire surface of the absorbent body 2, and the basis weight of the SAP is 160 g/m² or more on the entire surface of the absorbent body 2.

However, it is sufficient that the basis weight of SAP in at least a part of the absorbent body 2 is 160 g/m² or more.

In this manner, even in the case of decreasing the area of the absorbent body 2 in accordance with the body of the extremely-low-weight infant, the absorbent body 2 can absorb excrement reliably, making it possible to suppress the leakage of the excrement.

For example, in the case where excrement leaks and soils a bed sheet or the like when the absorbent pad 1 is put on a low-weight infant, it is necessary to change the bed sheet and concomitantly to move the body of the extremely-low-weight infant, causing a risk that the body of the extremely-low-weight infant is subject to the burden.

In contrast, when the basis weight of SAP is 160 g/m² or more, the urine excreted by the extremely-low-weight infant is more likely to be absorbed without leakage. Suppressing the leakage makes it possible to reduce the physical burden on the body of the extremely-low-weight infant.

The basis weight of SAP (absorbent polymer) of the absorbent body 2 can be measured by the following method.

First, taking out SAP of any mass from a to-be-measured absorbent body 2, neutralization titration is performed using 1 mol/L of hydrochloric acid, preparing a calibration curve, and the polymer coefficient of the SAP is calculated.

Next, a portion of the absorbent body 2 in a to-be-measured range is taken out from the absorbent body 2 as a sample, measuring the area of the sample viewed in the thickness direction and the mass of the sample (SAP + liquid-absorbent fiber).

Thereafter, the sample is put into ion exchange water in which an appropriate amount of sodium chloride is dissolved, and 1 mol/L of hydrochloric acid is injected 1 ml at a time, reading the amount of hydrochloric acid injected when the value of the pH meter for measuring the pH of the ion exchange water fluctuates significantly.

The product of the read value and the above polymer coefficient is the mass of SAP included in the sample.

The basis weight of SAP can be calculated by dividing the obtained value of the mass of the SAP by the above sample area.

In addition, the sealing portion 7 is formed throughout the entire periphery so as to frame the outer edge portion of the absorbent pad 1, but is provided so as not to overlap with the edge 1e of the top sheet 3 and the back sheet 6.

That is, the sealing portion 7 is provided extending along the edge 1e and spacing at a predetermined distance from the edge 1e of the absorbent pad. The edge 1e is a so-called dry edge.

The edge 1e of the absorbent pad 1 is a portion that is highly likely to come into contact with the body, for example, by being sandwiched between the legs of an extremely-low-weight infant when the absorbent pad is put on.

In the case where the edge 1e is hard due to the sealing portion 7, there is a risk of damaging the skin of the wearer (extremely-low-weight infant).

Therefore, in the present embodiment, making the edge 1e be a dry edge ensures the flexibility of the edge 1e, preventing the wearer's body from being injured when the absorbent pad is put on.

Usually, the absorbent pad 1 is not used by being attached to the underwear or disposable diaper of the wearer.

Therefore, it is desirable that in the non-skin-side surface of the absorbent pad 1 (non-skin-side surface of the back sheet 6), there is not provided an adhesive portion (prevention for displacement) which is for fixing the absorbent pad to the wearer's underwear or the like and/or which is for suppressing position shift.

This makes it easier to lay the absorbent pad 1 between the low-weight infant and the mat or the like when the absorbent pad 1 is put on.

In addition, it is possible to prevent the adhesive portion from sticking to the legs of the low-weight infant who takes a posture in which the legs are strongly bent into an M shape.

Therefore, nurses easily handle the absorbent pad 1.

In contrast, in the case where the absorbent pad 1 is provided with an adhesive portion, the adhesive portion can be used as a reference for distinguishing between the skin-side surface and the non-skin-side surface of the absorbent pad 1.

That is, in the case where the adhesive portion is not provided, it may be difficult to distinguish between the skin-side surface and the non-skin-side surface of the absorbent pad 1.

However, according to the absorbent pad 1 of the present embodiment, the skin-side surface and the non-skin-side surface of the absorbent pad 1 can be determined based on the design graphics 30 visually recognizable from the non-skin-side surface of the back sheet 6, making it possible to put on the absorbent pad 1 correctly.

In addition, as shown in FIG. 3, the absorbent pad 1 includes the liquid-permeable intermediate sheet 4 between the absorbent body 2 and the top sheet 3.

Therefore, the rigidity of the absorbent pad 1 increases compared with the case where the intermediate sheet 4 is not provided, making it easier to lay the absorbent pad 1 between the low-weight infant and the mat or the like.

In addition, in order to increase the rigidity of the absorbent pad 1, there is also a method of increasing the rigidity of the absorbent body 2 itself.

However, the absorbent pad 1 is more likely to be folded starting from the end of the absorbent body 2. Accordingly, when the legs of the low-weight infant are strongly bent into an M shape, the absorbent body 2 having high rigidity is sandwiched between the legs, causing a risk that the skin of the low-weight infant is injured.

Therefore, it is preferable to provide the intermediate sheet 4 on the absorbent pad 1 as described above.

It is preferable that the density of the intermediate sheet 4 (g/m³) is higher than the density of the top sheet 3 (g/m³).

Increasing the density of the intermediate sheet 4 increases the rigidity of the intermediate sheet 4, and also increases the rigidity of the absorbent pad 1.

This makes it easier to lay the absorbent pad 1 between the low-weight infant and the mat or the like. In addition, the ability for drawing the excrement from the top sheet 3 to the intermediate sheet 4 is increased, and the rewet rate is decreased. This can improve the sense of comfort of the absorbent pad 1.

The intermediate sheet 4 does not come into direct contact with the skin of the low-weight infant, and therefore it is less likely to damage the skin of the low-weight infant even when the intermediate sheet has higher rigidity than the top sheet 3.

The comparison of densities of the top sheet 3, the intermediate sheet 4 and the like may be performed by a well-known method.

In the present embodiment, the densities of the top sheet 3 and the intermediate sheet 4 are made uniform, but in the case where the densities of the top sheet and the intermediate sheet are not uniform, a plurality of points are measured, making a comparison based on the average density thereof.

As a method of comparing the densities, for example, portions of a predetermined size of the top sheet 3 and the intermediate sheet 4 are cut out as samples from the absorbent pad 1, measuring the area and mass of each sample, and calculating the basis weight (g/m²), which is the mass per unit area.

The density (g/m³) can also be calculated by measuring the thickness of each sample by the above method and dividing the basis weight (g/m²) of the sample by the thickness (m).

In addition, it is desirable that the intermediate sheet 4 includes a thermoplastic fiber.

As illustrated in FIG. 3, the intermediate sheet 4 is joined by the sealing portion 7 in a state of being sandwiched between the top sheet 3 and the back sheet 6 in the thickness direction.

Accordingly, since the intermediate sheet 4 includes the thermoplastic fiber, the intermediate sheet 4 is more likely to be fused between the top sheet 3 and the back sheet 6 when the heat sealing process is performed, making it possible to increase the joining strength.

The intermediate sheet 4 of the present embodiment is made of a spunbond nonwoven fabric which is formed by discharging melted thermoplastic polymer into a continuous long fiber.

Therefore, the joining strength of the sealing portion 7 can be sufficiently increased.

### Combined use of absorbent pad 1 and tape-type diaper 100

The absorbent pad 1 of the present embodiment can be used in combination with a tape-type disposable diaper 100 (hereinafter, also simply referred to as "diaper 100").

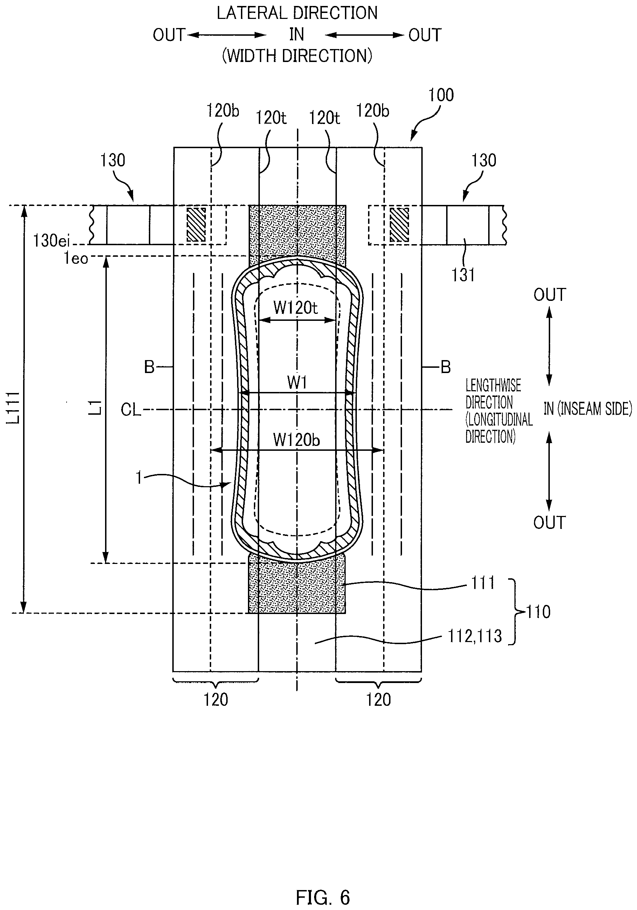

FIG. 6 is a diagram showing a state in which the absorbent pad 1 is placed on a skin-side surface of a diaper 100 in an unfolded and stretched state.

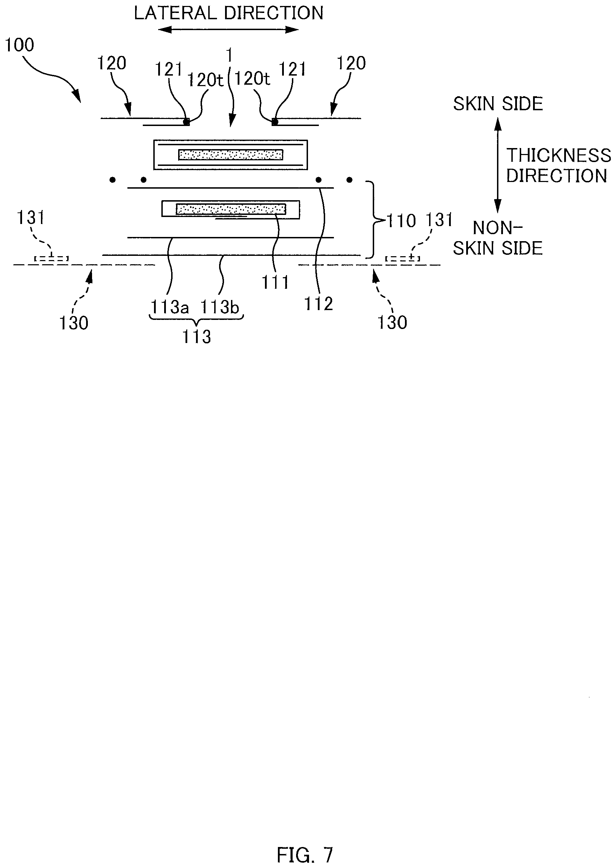

FIG. 7 is a schematic cross-sectional view taken along a line B-B of FIG. 6.

The diaper 100 is a general disposable diaper for a low-weight infant, and includes an absorbent main body 110, leakproof walls 120, and fastening tapes 130.

In addition, the diaper 100 has a lengthwise direction, a lateral direction, and a thickness direction orthogonal to each other in the unfolded state in FIG. 6.

The lengthwise direction corresponds to the longitudinal direction of the unfolded diaper 100, and corresponds to the longitudinal direction of the absorbent pad 1.

The lateral direction corresponds to the transverse direction of the unfolded diaper 100, and corresponds to the width direction of the absorbent pad 1.

The absorbent main body 110 includes: a liquid-absorbing absorbent core 111; a skin-side sheet 112 that is arranged on the skin side in the thickness direction with respect to the absorbent core 111; and a non-skin-side sheet 113 that is arranged on the non-skin side in the thickness direction with respect to the absorbent core 111. The absorbent main body 110 is a portion formed in a substantially rectangular shape elongated in the lengthwise direction.

The non-skin-side sheet 113 is constituted by a liquid-impermeable leakproof film 113a and an exterior sheet 113b overlaid on the non-skin side of the leakproof film 113a.

Further, the absorbent core 111 may be covered with a core-wrapping sheet.

The leakproof walls 120 are a pair of strip-shaped sheet members extending along the lengthwise direction and provided on the two lateral sides of the absorbent main body 110.

The two lengthwise-direction end portions and lateral outer end portions of the leakproof wall 120 are joined to the skin side of the absorbent main body 110 (skin-side sheet 112) by a leakproof-wall joining portion (not shown).

In addition, leakproof-wall elastic members 121 capable of stretching and contracting in the lengthwise direction are respectively provided at lateral inner ends 120t of the leakproof walls 120. When the diaper 100 is put on, the lateral inner ends120t of the leakproof walls 120 rises up toward the skin side of the wearer based on the stretchability exhibited by the leakproof-wall elastic member 121, serving as a leakproof wall that suppresses lateral leakage of excrement.

The fastening tape 130 is a pair of tape members provided on the lengthwise-direction back side of the absorbent main body 110 and protruding outward beyond two lateral end portions.

The lateral inner end portion of each fastening tape 130 is joined to the non-skin-side surface of the absorbent main body 110 (exterior sheet 113b). And the lateral outer end portion has a locking portion 131 on a skin-side surface thereof, which is constituted by a hook-and-loop fastener or the like.

When the diaper 100 is put on, the absorbent main body 110 is folded in half at the center position CL in the lengthwise direction, and the locking portion 131 is locked in a lengthwise-direction front region (non-skin-side surface) of the folded absorbent main body 110, forming a diaper 100 into an underpants shape (not shown).

In the present embodiment, the absorbent pad 1 can be attached to the diaper 100 for use.

When attaching the absorbent pad 1 to the diaper 100, the absorbent pad 1 is firstly arranged on the skin side of the absorbent main body 110 so that the lengthwise direction of the diaper 100 is aligned with the lengthwise direction of the absorbent pad 1 (see FIG. 6).

Then, the two widthwise end portions of the absorbent pad 1 are inserted into a space between the absorbent main body 110 and the leakproof walls 120 in the thickness direction (see FIG. 7).

As a result, the absorbent pad 1 is capable of being attached to and detached from the skin side of the diaper 100.

In this manner, when excretion is performed, the diaper 100 can be used repeatedly multiple times by changing the absorbent pad 1 that absorbed the excrement, making it possible to reduce the frequency of changing the diaper 100.

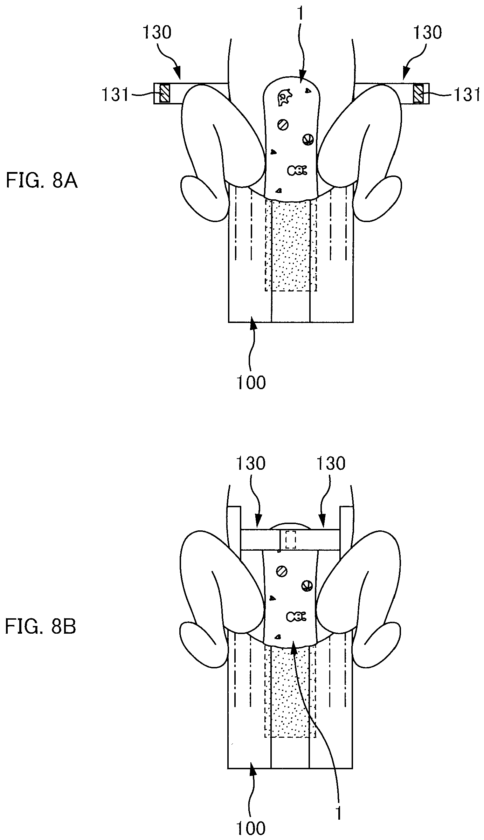

FIGS. 8A and 8B are diagrams for illustrating an aspect of use when the diaper 100 and the absorbent pad 1 are used in combination and put on an extremely-low-weight infant.

In the case where the absorbent pad is used for an extremely-low-weight infant, the absorbent pad 1 is used arranged on the skin-side surface of the diaper 100 in an overlapped manner, instead of attaching the absorbent pad to the diaper 100 by inserting two widthwise end portions of the absorbent pad 1 into the space between the absorbent main body 110 and the leakproof walls 120 of the diaper 100 as illustrated in FIGS. 6 and 7.

That is, as shown in FIG. 8A, by laying the unfolded diaper 100 under the absorbent pad 1, the excrement leaked from the absorbent pad 1 is captured by the tape type diaper 100.

As a result, the skin of the low-weight infant to which excrement adheres can be prevented soiling the mat and the like when the absorbent pad 1 is changed.

In addition, as described above, the frequency of changing the diaper 100 (for example, every half day or every day), which is more difficult to put on and take off than the absorbent pad 1, can be lower than the frequency of changing the absorbent pad 1 (for example, every 3 hours).

This can keep the low-weight infant rest, making it possible to reduce the burden on the nurse or the like.

Further, it is preferable that, as shown in FIG. 8B, the fastening tapes 130 of the diaper 100 is folded inward in the lateral direction (width direction) with respect to the absorbent pad 1 which is folded forward along the body of the low-weight infant, thereby locking the locking portion 131 to the non-skin-side surface (back sheet 6) of the absorbent pad 1.

Thus, it is possible to prevent the absorbent pad 1 from being unfolded in the longitudinal direction and to maintain a state in which the absorbent pad 1 is fitted along the body of the low-weight infant. Since the back sheet 6 of the present embodiment is formed of a nonwoven fabric, it is suitable for locking the locking portion 131 (hook-and-loop fastener) of the fastening tape 130 to it.

As shown in FIG. 6, it is desirable that the length (product length) L1 of the absorbent pad 1 in the longitudinal direction is smaller than a length L111 of the absorbent core 111 of the diaper 100 in the lengthwise direction.

In this manner even in the case where the excrement leaks outside in the longitudinal direction of the absorbent pad 1, the excrement is absorbed by the absorbent core 111 of the diaper 100 laid below the absorbent pad 1, making it more likely to prevent the excrement from leaking outside the diaper 100.

In addition, in FIG. 6, the lateral position of a rising base point where each leakproof wall 120 of the diaper 100 starts to rise up is defined as a position 120b. it is desirable that the lateral distance (gap) W120b between the rising base points 120b and 120b of the pair of left and right leakproof walls 120 is larger than the length (product width) W1 of the absorbent pad 1 in the width direction. Accordingly, the absorbent pad 1 can be inserted in and attached to the space between the absorbent main body 110 and the leakproof walls 120 in the thickness direction, without being compressed or folded in the width direction.

Therefore, it is possible to use the absorbent pad in combination with the diaper 100 without deteriorating the absorption performance of the absorbent pad 1.

In addition, it is desirable that in the diaper 100, a lateral distance (interval) W120t between the lateral inner ends 120t and 120t (that is, the rising tip ends of the leakproof walls 120) of the pair of left and right leakproof walls 120 is smaller than the length (product width) W1 of the absorbent pad 1 in the width direction.

Accordingly, the two widthwise end portions of the absorbent pad 1 are held so as to be covered by the tip end portions of the leakproof walls 120 from the skin side, making it less likely to displace the relative position of the absorbent pad 1 with respect to the diaper 100.

This can make it easier to fit the absorbent pad 1 to the body of a low-weight infant.

### Other Aspects of Absorbent Pad 1

Regarding the absorbent pad 1, in addition to the above-described aspect, the following aspects are possible.

### (Aspect 1)

An absorbent pad for a low-weight infant having a longitudinal direction, a width direction, and a thickness direction, which are orthogonal to each other, includes an absorbent body, a length of the absorbent pad in the longitudinal direction being 180 mm or less, a length of the absorbent pad in the width direction being less than 75 mm, edges of the absorbent pad on two longitudinal sides each having a curved portion.

As shown in FIGS. 1 and 2, the edges of the absorbent pad 1 on the two longitudinal sides are curved, softening the texture when the absorbent pad is put on and making it possible to reduce the burden on the body of a low-weight infant.

In addition, when the absorbent pad 1 is laid below the body of the sleeping low-weight infant, the absorbent pad 1 can be prevented from damaging the skin of low-weight infant.

### (Aspect 2)

The absorbent pad for a low-weight infant in which the edges of the absorbent pad on two widthwise sides each has a curved portion.

As shown in FIGS. 1 and 2, the edges of the absorbent pad 1 on the two widthwise sides are curved, softening the texture when the absorbent pad is put on and making it possible to reduce the burden on the body of a low-weight infant.

### (Aspect 3)

The absorbent pad for a low-weight infant in which the edge of the absorbent pad for a low-weight infant has a curved portion over the entire circumference.

As shown in FIGS. 1 and 2, the edge of the absorbent pad 1 is curved over the entire circumference, that is, the edge of the absorbent pad does not have a straight portion. This softens the texture when the absorbent pad is put on, making it possible to reduce the burden on the body of a low-weight infant.

### (Aspect 4)

The absorbent pad for a low-weight infant in which a narrow portion that is projected inward in the width direction is provided on two widthwise end portions.

As shown in FIG. 1, the absorbent pad 1 has a narrow portion 1c that is projected inward in the width direction at the longitudinal central region.

That is, the length (product width) W1 of the absorbent pad 1 in the width direction is smaller in the central part than in two longitudinal end portions.

As illustrated in FIG. 4A, when the absorbent pad 1 is put on, two widthwise end portions are sandwiched in the groin of the wearer.

Therefore, providing a narrow portion 1c in the two widthwise end portions makes it possible to reduce a load on the skin (groin) of the wearer.

In addition, since the narrow portion 1c is fitted so as to fit into the groin of the wearer, it is possible to suppress the position shift of the absorbent pad 1 when the absorbent pad is put on.

### (Aspect 5)

The absorbent pad for a low-weight infant in which the narrow portion is provided at the longitudinal central portion.

The longitudinal central portion is a portion positioned at the crotch (groin) of the wearer when the absorbent pad 1 is put on. Accordingly, the product width W1 in this portion is narrow, making it more likely to fit the absorbent pad 1 to the body of the wearer.

In addition, the absorbent pad 1 has a shape in which its front and back portions are substantially symmetrical with respect to the narrow portion 1c located in the longitudinal central portion. Therefore, the absorbent pad 1 can be placed facing backward, and it s less likely to cause a problem that the fitting property deteriorates due to incorrectly using the front and back portions in the longitudinal direction.

### (Aspect 6)

The absorbent pad for a low-weight infant in which a value obtained by dividing a minimum value of the length in the width direction by a maximum value of the length in the width direction is 0.9 or more.

In FIG. 1, assuming that the minimum value of the width-direction length of the portion in which the product width W1 is the shortest is defined as a length W1s and the maximum value of the width-direction length of the portion in which the product width W1 is the longest is defined as a length W1l, the smaller the value of W1s/W1l, the larger the constriction.

An extremely-low-weight infant whose skin is weak is also a target wearer of the absorbent pad 1 of the present embodiment. In order to minimize the irritation given to the skin of the extremely-low-weight infant as much as possible, it is desirable that the constriction is small.

That is, it is desirable that the curve of the edge portion (outer edge portion) of the absorbent pad 1 is as smooth as possible.

Specifically, if W1s/W1l ≥ 0.9, the irritation given to the skin of an extremely-low-weight infant can be minimized while suppressing the deterioration of the fitting property.

In the present embodiment, since W1s = 55 mm and W1s = 60 mm, the value of W1s/W1l is about 0.917.

Therefore, a configuration is established which makes it easier to achieve both fitting property and safety during wearing.

### (Aspect 7)

The absorbent pad for a low-weight infant in which a length of the absorbent body in the width direction is 39 mm or less.

The crotch width of an extremely-low-weight infant having a weight of less than 1000 g is about 35 mm.

Therefore, if a width W2 of the absorbent body 2 having high rigidity is too wide, the absorbent body 2 is not fitted between the legs of the extremely-low-weight infant when the absorbent pad is put on, making it more likely to deteriorate the fitting property.

In addition, when such an absorbent body 2 is forcibly put on the extremely-low-weight infant, there is a risk of injuring both legs of the infant.

Therefore, in the present embodiment, the length (width) W2 of the absorbent body 2 in the width direction is set to 39 mm or less.

With such a size, the absorbent pad 1 can be comfortably fitted between the legs of the low-weight infant in a posture in which the legs are strongly bent into an M shape.

### (Aspect 8)

The absorbent pad for a low-weight infant in which the absorbent body has an absorbent-body narrow portion that is projected inward in the width direction, in two widthwise end portions of the absorbent body.

It is described above that the absorbent pad 1 of the present embodiment has the narrow portion 1c in which the width W1 in the width direction is narrowed, and also it is desirable that the absorbent body 2 itself has an absorbent-body narrow portion 2c in which the width W2. in the width direction is narrowed.

In this manner, when the absorbent body 2 having high rigidity is sandwiched in the groin of the extremely-low-weight infant during putting on the absorbent pad 1, it is possible to reduce a load on the skin (groin) of the extremely-low-weight infant.

### (Aspect 9)

The absorbent pad for a low-weight infant in which the length of the absorbent body in the width direction is constant.

On the other hand, as described above, in the case where the length (width) W2 of the absorbent body 2 in the width direction is 39 mm or less, the absorbent-body narrow portion 2c does not necessarily have to be provided.

That is, the width W2 of the absorbent body 2 in the width direction may be constant.

According to such an absorbent body 2, since the absorbent-body narrow portion 2c is not provided, it is possible to make larger the area of the absorbent body 2 by the area of the absorbent-body narrow portion. This makes it possible to increase the absorption capacity for excrement such as urine.

### (Aspect 10)

The absorbent pad for a low-weight infant in which the value obtained by dividing the minimum value of the width-direction length of the absorbent pad by the maximum value of the width-direction length of the absorbent pad is equal to or greater than a value obtained by dividing a minimum value of the width-direction length of the absorbent body by a maximum value of the width-direction length of the absorbent body.

In the case where the absorbent-body narrow portion 2c is provided in the absorbent body 2, when the minimum value of the width-direction length (width) of the portion in which the absorbent body width W2 is the shortest is defined as a width W2s, and the maximum value of the width-direction length (width) of the portion in which the absorbent body width W2 is the longest is defined as a width W2l, the value of W1s/W1l described above is equal to or greater than a value of W2s/W2l in the present embodiment.

That is, the size of the constriction (narrow portion 1c) at the edge portion (outer edge portion) of the absorbent pad 1 is equal to or less than the size of the constriction (absorbent-body narrow portion 2c) at the edge portion (outer edge portion) of the absorbent body 2.

In other words, the gentleness of the curve of the outer edge portion of the absorbent pad 1 is equal to or less than the gentleness of the curve of the outer edge portion of the absorbent body 2.

This makes gentler the curve of the outer edge portion of the absorbent pad 1 that comes into direct contact with the skin of the extremely-low-weight infant when putting on, so that the skin of the extremely-low-weight infant can be made less likely to be damaged.

### (Aspect 11)

The absorbent pad for a low-weight infant in which the value obtained by dividing the minimum value of the width-direction length of the absorbent pad by the maximum value of the width-direction length of the absorbent pad is smaller than the value obtained by dividing the minimum value of the width-direction length of the absorbent body by the maximum value of the width-direction length of the absorbent body.

In contrast, the value of W1s/W1l described above may be smaller than the value of W2s/W2l.

In this case, the constriction at the edge portion (outer edge portion) of the absorbent body 2 (absorbent-body narrow portion 2c) is smaller than the constriction at the edge portion (outer edge portion) of the absorbent pad 1 (narrow portion 1c).

That is, the narrow portion 2c of the absorbent body becomes smaller, and the width-direction length (width) W2. of the absorbent body 2 is close to a fixed value.

This can increase the area of the absorbent body 2, making it possible to increase the absorption capacity for excrement such as urine.

### (Aspect 12)

The absorbent pad for a low-weight infant in which at the longitudinal center position, a value obtained by dividing the length of the absorbent body in the width direction by the length of the absorbent pad for a low-weight infant in the width direction is 0.7 or less.

In FIG. 1, the smaller the value W2/W1 which is obtained by dividing the width W2 (W2s) of the absorbent body 2 at the longitudinal center position CL by the width W1 (W1s) of the absorbent pad 1 at the longitudinal center position, the larger the difference between the width W2. of the absorbent body 2 and the width W1 of the absorbent pad 1.

That is, a distance dw12 (see FIG. 1) between a widthwise outer end of the absorbent pad 1 and a widthwise outer end of the absorbent body 2 becomes larger.

In the case where the value W2/W1 is large (close to 1), the distance cMr12 is close to zero.

In this case, when the absorbent pad 1 is sandwiched in the groin of the wearer during wearing, the edge of the absorbent body 2 having high rigidity in the width direction easily come into contact with both legs of the wearer.

On the other hand, when the value of W2/W1 is small, when the absorbent pad 1 is sandwiched in the groin of the wearer during wearing, the portion of the distance dw12 (that is, the portion having low rigidity) in which the absorbent body 2 is not provided is deformed so as to be crushed in the width direction and functions like a cushion.

As a result, the groin of the wearer are easily prevented from being damaged by the edge (edge) of the absorbent body 2 having high rigidity.

Therefore, in order to ensure the cushioning region, it is desirable that the value of W2/W1 is 0.7 or less.

In the present embodiment, W2/W1 = 35 mm/55 mm = 0.636, and a region (a region of the distance dw12) that functions as a cushion can be sufficiently ensured.

### (Aspect 13)

The absorbent pad for a low-weight infant in which a difference between a thickness-direction thickness of a portion of in the absorbent pad in which the absorbent body is provided and a thickness-direction thickness of a portion of in the absorbent pad in which the absorbent body is not provided is 1.5 mm or less.

In the absorbent pad 1, a portion in which the absorbent body 2 is provided (for example, a portion A2 in FIG. 1) has a thickness larger than a portion in which the absorbent body 2 is not provided (for example, a portion A6 in FIG. 1), by the thickness of the absorbent body 2.

When this difference in thickness is large, a step in the thickness direction is formed on the surface of the absorbent pad 1. In this case, when laying the absorbent pad 1 below the body of the low-weight infant, there is a risk of damaging the skin of the low-weight infant due to the step.

Therefore, in the present embodiment, the difference in thickness between the portion in which the absorbent body 2 is provided and the portion in which the absorbent body 2 is not provided is set to 1.5 mm or less, not generating a large step on the surface of the absorbent pad 1.

As a result, it is possible to prevent the skin of a low-weight infant from being damaged.

The thickness is measured using PEACOCK FFD-1 manufactured by OZAKI MFG.CO., LTD.. With adjusting the pressurization by the terminal to 165 g/cm², a plurality of points (for example, 20 points) in the portion of the absorbent pad 1 in which the absorbent body 2 is provided are measured, and the average value is calculated.

Under the same conditions, the thicknesses of the plurality of points in the portion of the absorbent pad 1 in which the absorbent body 2 is not provided are measured, and the average value is obtained. As a result of the measurement, the thickness (average value) of the portion in which the absorbent body 2 was provided was 1.795 mm.

On the other hand, the thickness (average value) of the portion in which the absorbent body 2 was not provided was 0.494 mm.

Therefore, in the present embodiment, it has been confirmed that the difference between the thickness of the portion in which the absorbent body 2 is provided and the thickness of the portion in which the absorbent body 2 is not provided is 1.301 mm, and a large step is not generated.

### (Aspect 14)

The absorbent pad for a low-weight infant being capable of being attached to and detached from a skin side of an absorbent main body of a disposable diaper, the disposable diaper having a lengthwise direction, a lateral direction, and a thickness direction, which are orthogonal to each other, the disposable diaper including: the absorbent main body including an absorbent core; a pair of leakproof walls that are provided on two lateral sides of the absorbent main body and are capable of rising up toward the skin side in the thickness direction; and a pair of fastening tapes that protrude outward beyond two lateral end portions of the absorbent main body, wherein the absorbent pad for a low-weight infant does not have a folding line for folding the absorbent pad in the longitudinal direction, and the disposable diaper has a folding line for folding the diaper in the lengthwise direction.

The diaper 100 used in combination with the absorbent pad 1 has a substantially rectangular shape that is elongated in the lengthwise direction as shown in FIG. 6, in the stretched state. Accordingly, the diaper is folded in the lengthwise direction and distributed in a compact shape on the market. Therefore, the folding line for folding the diaper 100 in the lengthwise direction is provided at a predetermined position (for example, the center position CL) in the lengthwise direction.

Therefore, when the diaper 100 is put on, the diaper 100 is likely to be folded along the folding line. On the other hand, as described above, the absorbent pad 1 is not provided with a folding line for folding the absorbent pad in the longitudinal direction.

When the absorbent pad 1 and the diaper 100 are used in combination and put on an extremely-low-weight infant, the absorbent pad 1 can be covered from the non-skin side by the diaper 100 which is folded along the crotch portion of the extremely-low-weight infant, while the absorbent pad 1 not having the folding line is perfectly fitted to the crotch portion of the low-weight infant (see FIGS. 4A and 4B).

Therefore, the position of the absorbent pad 1 is less likely to be shifted with respect to the body of the extremely-low-weight infant, and it is possible to easily suppress the leakage of excrement.

### (Aspect 15)

The absorbent pad for a low-weight infant in which the fastening tape of the disposable diaper is lockable to a non-skin-side surface of the absorbent pad in a state where the absorbent pad is attached to the skin side of the absorbent main body of the disposable diaper.

As illustrated in FIG. 8B, when the absorbent pad 1 is put on the extremely-low-weight infant, the fastening tape 130 (locking portion 131) of the diaper 100 can be locked to the non-skin-side surface of the absorbent pad 1 which is arranged along the body of the low-weight infant.

This makes it possible to prevent the absorbent pad 1 from being unfolded in the longitudinal direction, making it easier to fit the absorbent pad 1 to the body of the low-weight infant.

### (Aspect 16)

The absorbent pad for a low-weight infant in which when the absorbent pad and the disposable diaper overlap in the thickness direction in a state where the longitudinal center position of the absorbent pad and the lengthwise center position of the disposable diaper are aligned, a longitudinal outer end of the absorbent pad is located inside in the lengthwise direction with respect to lengthwise inner ends of the fastening tapes.

As shown in FIG. 6, in the case where both the absorbent pad and the diaper overlap in the thickness direction in a state where the longitudinal center position of the absorbent pad 1 and the lengthwise center position of the diaper 100 are aligned, the longitudinal outer end 1 eo of the absorbent pad 1 is located inside in the lengthwise direction than an lengthwise inner end 130ei of the fastening tape 130.

That is, in the lengthwise direction, the fastening tape 130 and the absorbent pad 1 do not overlap.

When the absorbent pad 1 and the diaper 100 are used in combination and put on an extremely-low-weight infant, in order to lock the fastening tape 130 to the non-skin-side surface of the absorbent pad 1 as shown in FIG. 8B from such a state, it is necessary to overlap the absorbent pad 1 in the lengthwise direction with the fastening tape 130 on the stomach side of the extremely-low-weight infant.

Therefore, a user (a person who puts the absorbent pad 1 on the extremely-low-weight infant) performs an operation of fitting the absorbent pad 1 to the crotch of the extremely-low-weight infant while gently pulling the absorbent pad 1 toward the stomach side of the extremely-low-weight infant. This makes it easier to bring the absorbent pad 1 into close contact with the crotch portion of the extremely-low-weight infant, making it possible to enhance the fitting property.

### Other Embodiments

Although the above embodiment of the present invention has been described, but the above-described embodiment is intended to facilitate the understanding of the present invention and is not intended to limit the interpretation of the present invention.

### [REFERENCE SIGNS LIST]

1 absorbent pad (absorbent pad for a low-weight infant), 1c narrow portion, 1e edge, 1 eo outer side end, 2 absorbent body, 2a absorbent core, 2b core-wrapping sheet, 2c absorbent-body narrow portion, 3 top sheet, 4 intermediate sheet, 5 leakproof sheet, 6 back sheet, 7 sealing portion, 10 adhesive, 30 design pattern, 50 absorbent pad (comparative example), 100 diaper (tape type), 110 absorbent main body, 111 absorbent core, 112 skin-side sheet, 113 non-skin-side sheet, 113a leakproof film, 113b exterior sheet, 120 leakproof wall, 120b position (standing base point), 120t inner side end, 121 leakproof-wall elastic member, 130 fastening tape, 130ei inner side end, 131 locking portion, l1 length (product length of absorbent pad 1), w1 length (product width of absorbent pad 1)

## Claims

1. An absorbent pad (1) for a low-weight infant having a longitudinal direction, a width direction, and a thickness direction, that are orthogonal to each other,
the absorbent pad (1) comprising:
an absorbent body (2),
a length (L1) of the absorbent pad (1) in the longitudinal direction being 180 mm or less,
a length (W1) of the absorbent pad (1) in the width direction being less than 75 mm,
wherein
the absorbent pad (1) further comprises a back sheet (6) that is arranged on a non-skin side in the thickness direction with respect to the absorbent body (2), and
the back sheet (6) is made of a nonwoven fabric, wherein
the absorbent pad (1) further comprises:
a top sheet (3) that is arranged on a skin side in the thickness direction with respect to the absorbent body (2), and
a sealing portion (7) in which at least a part of the top sheet (3) is joined to the back sheet (6) in the thickness direction, and wherein
the sealing portion (7) is provided extending along an outer edge of the absorbent pad (1) for a low-weight infant and spacing from the outer edge at a predetermined distance.

2. The absorbent pad for a low-weight infant according to claim 1, wherein
an elastic member is not provided in a region outside the absorbent body (2) in the width direction.

3. The absorbent pad for a low-weight infant according to claim 1 or 2, wherein
the absorbent pad (1) does not have a folding line for folding the absorbent pad (1) in the longitudinal direction.

4. The absorbent pad (1) for a low-weight infant according to claim 1 or 2, wherein
the absorbent body (2) includes an absorbent polymer, and
the absorbent pad (1) has a portion that is a part of an entire periphery of the sealing portion (7) and that is not peeled off even when the absorbent polymer swells.

5. The absorbent pad (1) for a low-weight infant according to any one of claims 1 to 4, wherein
the absorbent body (2) includes an absorbent polymer, and
a basis weight of the absorbent polymer in the absorbent body (2) is 160 g/m2 or more.

6. The absorbent pad (1) for a low-weight infant according to any one of claims 1 to 5, wherein
the absorbent pad (1) does not include an adhesive portion for fixing the absorbent pad (1) to a underwear of a wearer.

7. The absorbent pad (1) for a low-weight infant according to any one of claims 1 to 6, wherein
the absorbent pad (1) further comprises:
a top sheet (3) that is arranged on a skin side in the thickness direction with respect to the absorbent body (2), and
an intermediate sheet that is arranged between the absorbent body (2) and the top sheet (3) in the thickness direction.

8. The absorbent pad (1) for a low-weight infant according to claim 7, wherein
a density of the intermediate sheet is higher than a density of the top sheet.

9. The absorbent pad (1) for a low-weight infant according to claim 7 or 8, wherein
the intermediate sheet includes a thermoplastic fiber.

10. The absorbent pad (1) for a low-weight infant according to any one of claims 1 to 9, wherein
the absorbent pad (1) is attached to a skin side of an absorbent main body of a disposable diaper,
the disposable diaper (100) having a lengthwise direction, a lateral direction, and a thickness direction, that are orthogonal to each other,
the disposable diaper (100) including:
the absorbent main body including an absorbent core,
a pair of leakproof walls that are provided on two lateral sides of the absorbent main body and are capable of rising up toward the skin side in the thickness direction; and
a pair of fastening tapes that protrude outward beyond two lateral end portions of the absorbent main body.

11. The absorbent pad (1) for a low-weight infant according to claim 10, wherein
a length of the absorbent pad (1) for a low-weight infant in the longitudinal direction is smaller than a length of the absorbent core in the lengthwise direction.

12. The absorbent pad (1) for a low-weight infant according to claim 10 or 11, wherein
a length of the absorbent pad (1) for a low-weight infant in the width direction is smaller than a lateral length between rising base points of the pair of leakproof walls,
the rising base point being a point where each of the pair of leakproof walls starts to rise up toward the skin side.

13. The absorbent pad (1) for a low-weight infant according to any one of claims 10 to 12, wherein
a length of the absorbent pad (1) for a low-weight infant in the width direction is larger than a lateral length between peaks of the pair of leakproof walls.

## Patentansprüche

1. Absorbierendes Kissen (1) für ein leichtgewichtiges Kleinkind mit einer Längsrichtung, einer Breitenrichtung und einer Dickenrichtung, die orthogonal zueinander verlaufen,
wobei das absorbierende Kissen (1) umfasst:
einen absorbierenden Körper (2),
eine Länge (L1) des absorbierenden Kissens (1) in Längsrichtung, die sich auf 180 mm oder weniger beläuft,
eine Länge (W1) des absorbierenden Kissens (1) in Breitenrichtung, die sich auf weniger als 75 mm beläuft,
wobei
das absorbierende Kissen (1) ferner eine Rückseitenfolie (6) umfasst, die auf einer Nicht-Haut-Seite in Dickenrichtung in Bezug auf den absorbierenden Körper (2) angeordnet ist, und
die Rückseitenfolie (6) aus einem Vliesstoff hergestellt ist, wobei
das absorbierende Kissen (1) ferner umfasst:
eine obere Folie (3), die auf einer Hautseite in Dickenrichtung in Bezug auf den absorbierenden Körper (2) angeordnet ist, und
einen Dichtungsabschnitt (7), in dem wenigstens ein Teil der oberen Folie (3) in Dickenrichtung mit der Rückseitenfolie (6) verbunden ist, und wobei
der Dichtungsabschnitt (7) so bereitgestellt ist, dass er sich entlang eines Außenrands des absorbierenden Kissens (1) für ein leichtgewichtiges Kleinkind erstreckt und in einem vorbestimmten Abstand von dem Außenrand beabstandet ist.

2. Absorbierendes Kissen für ein leichtgewichtiges Kleinkind nach Anspruch 1,
wobei
ein elastisches Element nicht in einem Bereich außerhalb des absorbierenden Körpers (2) in Breitenrichtung bereitgestellt ist.

3. Absorbierendes Kissen für ein leichtgewichtiges Kleinkind nach Anspruch 1 oder 2, wobei
das absorbierende Kissen (1) keine Faltlinie zum Falten des absorbierenden Kissens (1) in Längsrichtung aufweist.

4. Absorbierendes Kissen (1) für ein leichtgewichtiges Kleinkind nach Anspruch 1 oder 2, wobei
der absorbierende Körper (2) ein absorbierendes Polymer beinhaltet, und
das absorbierende Kissen (1) einen Abschnitt aufweist, der ein Teil eines Gesamtumfangs des Dichtungsabschnitts (7) ist und nicht einmal dann abgezogen wird, wenn das absorbierende Polymer quillt.

5. Absorbierendes Kissen (1) für ein leichtgewichtiges Kleinkind nach einem der Ansprüche 1 bis 4, wobei
der absorbierende Körper (2) ein absorbierendes Polymer beinhaltet, und
ein Grundgewicht des absorbierenden Polymers in dem absorbierenden Körper (2) 160 g/m² oder mehr beträgt.

6. Absorbierendes Kissen (1) für ein leichtgewichtiges Kleinkind nach einem der Ansprüche 1 bis 5, wobei
das absorbierende Kissen (1) keinen Klebeabschnitt zum Fixieren des absorbierenden Kissens (1) an einer Unterwäsche eines Trägers aufweist.

7. Absorbierendes Kissen (1) für ein leichtgewichtiges Kleinkind nach einem der Ansprüche 1 bis 6, wobei
das absorbierende Kissen (1) ferner umfasst:
eine obere Folie (3), die auf einer Hautseite in Dickenrichtung in Bezug auf den absorbierenden Körper (2) angeordnet ist, und
eine Zwischenfolie, die zwischen dem absorbierenden Körper (2) und der oberen Folie (3) in Dickenrichtung angeordnet ist.

8. Absorbierendes Kissen (1) für ein leichtgewichtiges Kleinkind nach Anspruch 7, wobei
eine Dichte der Zwischenfolie höher als eine Dichte der oberen Folie ist.

9. Absorbierendes Kissen (1) für ein leichtgewichtiges Kleinkind nach Anspruch 7 oder 8, wobei
die Zwischenfolie eine thermoplastische Faser beinhaltet.

10. Absorbierendes Kissen (1) für ein leichtgewichtiges Kleinkind nach einem der Ansprüche 1 bis 9, wobei
das absorbierende Kissen (1) an einer Hautseite eines absorbierenden Hauptkörpers einer Einwegwindel angebracht ist, wobei die Einwegwindel (100) eine Längenrichtung, eine Lateralrichtung und eine Dickenrichtung aufweist, die orthogonal zueinander verlaufen,
wobei die Einwegwindel (100) beinhaltet:
den absorbierenden Hauptkörper mit einem absorbierenden Kern,
ein Paar von auslaufsicheren Wänden, die auf zwei Lateralseiten des absorbierenden Hauptkörpers bereitgestellt und in der Lage sind, sich hin zu der Hautseite in Dickenrichtung zu erheben; und
ein Paar von Befestigungsbändern, die nach außen über zwei laterale Endabschnitte des absorbierenden Hauptkörpers hinaus vorstehen.

11. Absorbierendes Kissen (1) für ein leichtgewichtiges Kleinkind nach Anspruch 10, wobei
eine Länge des absorbierenden Kissens (1) für ein leichtgewichtiges Kleinkind in Längsrichtung kürzer als eine Länge des absorbierenden Kerns in Längenrichtung ist.

12. Absorbierendes Kissen (1) für ein leichtgewichtiges Kleinkind nach Anspruch 10 oder 11, wobei
eine Länge des absorbierenden Kissens (1) für ein leichtgewichtiges Kleinkind in Breitenrichtung kürzer als eine Laterallänge zwischen sich erhebenden Basispunkten des Paares von auslaufsicheren Wänden ist,
wobei der sich erhebende Basispunkt ein Punkt ist, an dem sich jede des Paares von auslaufsicheren Wänden hin zu der Hautseite zu erheben beginnt.

13. Absorbierendes Kissen (1) für ein leichtgewichtiges Kleinkind nach einem der Ansprüche 10 bis 12, wobei
eine Länge des absorbierenden Kissens (1) für ein leichtgewichtiges Kleinkind in Breitenrichtung länger als eine Laterallänge zwischen Spitzen des Paares von auslaufsicheren Wänden ist.

## Revendications

1. Garniture absorbante (1) pour bébé en insuffisance pondérale ayant une direction longitudinale, une direction de la largeur et une direction de l'épaisseur, qui sont orthogonales l'une à l'autre,
la garniture absorbante (1) comprenant :
un corps absorbant (2),
une longueur (L1) de la garniture absorbante (1) dans la direction longitudinale uétant de 180 mm ou moins,
une longueur (W1) de la garniture absorbante (1) dans la direction de la largeur étant inférieure à 75 mm,
dans laquelle
la garniture absorbante (1) comprend également une feuille arrière (6) qui est agencée sur un côté non cutané dans la direction de l'épaisseur par rapport au corps absorbant (2), et
la feuille arrière (6) est constituée d'un tissu non tissé, dans laquelle la garniture absorbante (1) comprend également :
une feuille supérieure (3) qui est agencée sur un côté cutané dans la direction de l'épaisseur par rapport au corps absorbant (2), et
une partie d'étanchéité (7) dans laquelle au moins une partie de la feuille supérieure (3) est jointe à la feuille arrière (6) dans la direction de l'épaisseur, et dans laquelle
la partie d'étanchéité (7) est prévue s'étendant le long d'un bord externe de la garniture absorbante (1) pour bébé en insuffisance pondérale et espacée du bord externe à une distance prédéterminée.

2. Garniture absorbante pour bébé en insuffisance pondérale selon la revendication 1, dans laquelle
un élément élastique n'est pas prévu dans une région à l'extérieur du corps absorbant (2) dans la direction de la largeur.

3. Garniture absorbante pour bébé en insuffisance pondérale selon la revendication 1 ou 2, dans laquelle
la garniture absorbante (1) n'a pas de ligne de pliage pour plier la garniture absorbante (1) dans la direction longitudinale.

4. Garniture absorbante (1) pour bébé en insuffisance pondérale selon la revendication 1 ou 2, dans laquelle
le corps absorbant (2) comprend un polymère absorbant, et
la garniture absorbante (1) a une partie qui fait partie d'une périphérie entière de la partie d'étanchéité (7) et qui n'est pas décollée même lorsque le polymère absorbant gonfle.

5. Garniture absorbante (1) pour bébé en insuffisance pondérale selon l'une quelconque des revendications 1 à 4, dans laquelle
le corps absorbant (2) comprend un polymère absorbant, et
un poids de base du polymère absorbant dans le corps absorbant (2) est de 160 g/m2 ou plus.

6. Garniture absorbante (1) pour bébé en insuffisance pondérale selon l'une quelconque des revendications 1 à 5, dans laquelle
la garniture absorbante (1) ne comprend pas de partie adhésive pour fixer la garniture absorbante (1) à un sous-vêtement d'un porteur.

7. Garniture absorbante (1) pour bébé en insuffisance pondérale selon l'une quelconque des revendications 1 à 6, dans laquelle
la garniture absorbante (1) comprend également :
une feuille supérieure (3) qui est agencée sur un côté cutané dans la direction de l'épaisseur par rapport au corps absorbant (2), et
une feuille intermédiaire qui est agencée entre le corps absorbant (2) et la feuille supérieure (3) dans la direction de l'épaisseur.

8. Garniture absorbante (1) pour bébé en insuffisance pondérale selon la revendication 7, dans laquelle
une densité de la feuille intermédiaire est supérieure à une densité de la feuille supérieure.

9. Garniture absorbante (1) pour bébé en insuffisance pondérale selon la revendication 7 ou 8, dans laquelle
la feuille intermédiaire comprend une fibre thermoplastique.

10. Garniture absorbante (1) pour bébé en insuffisance pondérale selon l'une quelconque des revendications 1 à 9, dans laquelle
la garniture absorbante (1) est fixée à un côté cutané d'un corps principal absorbant d'une couche jetable, la couche jetable (100) ayant une direction longitudinale, une direction latérale et une direction de l'épaisseur, qui sont orthogonales l'une à l'autre,
la couche jetable (100) comprenant :
le corps principal absorbant comprenant un noyau absorbant,
une paire de parois étanches qui sont prévues sur deux côtés latéraux du corps principal absorbant et sont capables de s'élever vers le côté cutané dans la direction de l'épaisseur ; et
une paire de rubans de fixation qui font saillie vers l'extérieur au-delà de deux parties d'extrémité latérales du corps principal absorbant.

11. Garniture absorbante (1) pour bébé en insuffisance pondérale selon la revendication 10, dans laquelle
une longueur de la garniture absorbante (1) pour bébé en insuffisance pondérale dans la direction longitudinale est inférieure à une longueur du noyau absorbant dans la direction longitudinale.

12. Garniture absorbante (1) pour bébé en insuffisance pondérale selon la revendication 10 ou 11, dans laquelle
une longueur de la garniture absorbante (1) pour bébé en insuffisance pondérale dans la direction de la largeur est inférieure à une longueur latérale entre des points de base ascendants de la paire de parois étanches,
le point de base ascendant étant un point où chacune de la paire de parois étanches commence à s'élever vers le côté cutané.

13. Garniture absorbante (1) pour bébé en insuffisance pondérale selon l'une quelconque des revendications 10 à 12, dans laquelle
une longueur de la garniture absorbante (1) pour bébé en insuffisance pondérale dans la direction de la largeur est supérieure à une longueur latérale entre les sommets de la paire de parois étanches.
